(19) Europäisches Patentamt
European Patent Office
Office européen des brevets

(11) EP 3 083 973 B1

(12) # FASCICULE DE BREVET EUROPEEN

(45) Date de publication et mention de la délivrance du brevet:
**08.05.2019 Bulletin 2019/19**

(21) Numéro de dépôt: **14830829.9**

(22) Date de dépôt: **18.12.2014**

(51) Int Cl.:
***C12P 7/64*** *(2006.01)*

(86) Numéro de dépôt international:
**PCT/FR2014/053430**

(87) Numéro de publication internationale:
**WO 2015/092301 (25.06.2015 Gazette 2015/25)**

(54) **PROCEDE D'ENRICHISSEMENT EN DHA DE LA BIOMASSE DE MICROALGUES DU GENRE TRAUSTOCHYTRIUM**

DHA BEREICHERUNGSVERFAHREN VON TRAUSTOCHYTRIUM SPECIES MIKROALGEN

DHA ENRICHMENT PROCESS OF THE TRAUSTOCHYTRIUM SPECIES MICROALGAE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.12.2013 FR 1362962**

(43) Date de publication de la demande:
**26.10.2016 Bulletin 2016/43**

(73) Titulaire: **Roquette Frères**
**62136 Lestrem (FR)**

(72) Inventeur: **CAULIER, Bernard**
**F-59273 Fretin (FR)**

(74) Mandataire: **Cabinet Plasseraud**
**66, rue de la Chaussée d'Antin**
**75440 Paris Cedex 09 (FR)**

(56) Documents cités:
**WO-A1-01/54510** **WO-A1-2005/021735**
**US-A1- 2009 209 014**

- **CHANG GUIFANG ET AL: "Fatty acid shifts and metabolic activity changes ofSchizochytriumsp. S31 cultured on glycerol", BIORESOURCE TECHNOLOGY, ELSEVIER BV, GB, vol. 142, 16 mai 2013 (2013-05-16), pages 255-260, XP028576201, ISSN: 0960-8524, DOI: 10.1016/J.BIORTECH.2013.05.030**
- **LI JUAN ET AL: "Comparative metabolomics analysis of docosahexaenoic acid fermentation processes by Schizochytrium sp. under different oxygen availability conditions.", OMICS : A JOURNAL OF INTEGRATIVE BIOLOGY MAY 2013, vol. 17, no. 5, mai 2013 (2013-05), pages 269-281, XP002728860, ISSN: 1557-8100**
- **LIANG QU ET AL: "Batch, fed-batch and repeated fed-batch fermentation processes of the marine thraustochytrid Schizochytrium sp. for producing docosahexaenoic acid", BIOPROCESS AND BIOSYSTEMS ENGINEERING, vol. 36, no. 12, 15 décembre 2013 (2013-12-15), pages 1905-1912, XP055136084, ISSN: 1615-7591, DOI: 10.1007/s00449-013-0966-7**
- **ZHANG LIN ET AL: "Improving docosahexaenoic acid productivity ofSchizochytriumsp. by a two-stage AEMR/shake mixed culture mode", BIORESOURCE TECHNOLOGY, ELSEVIER BV, GB, vol. 142, 24 mai 2013 (2013-05-24), pages 719-722, XP028576237, ISSN: 0960-8524, DOI: 10.1016/J.BIORTECH.2013.05.072**
- **QU LIANG ET AL: "Scale-up of docosahexaenoic acid production in fed-batch fermentation bySchizochytriumsp. based on volumetric oxygen-transfer coefficient", BIOCHEMICAL ENGINEERING JOURNAL, vol. 77, 15 mai 2013 (2013-05-15), pages 82-87, XP028682172, ISSN: 1369-703X, DOI: 10.1016/J.BEJ.2013.05.011**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 083 973 B1

- GUIFANG CHANG ET AL: "The relationship of oxygen uptake rate and kLa with rheological properties in high cell density cultivation of docosahexaenoic acid by Schizochytrium sp. S31", BIORESOURCE TECHNOLOGY, vol. 152, 1 janvier 2014 (2014-01-01), pages 234-240, XP055136035, ISSN: 0960-8524, DOI: 10.1016/j.biortech.2013.11.002

**Description**

La présente invention se rapporte à un nouveau procédé fermentaire d'enrichissement en acide docosahexaénoïque (ou DHA) de la biomasse de microalgues du genre *Thraustochytrium,* plus particulièrement *Schizochytrium sp ou Schizochytrium mangrovei,* ainsi que de l'huile extraite de cette biomasse de microalgue.
Les lipides constituent une des trois grandes familles de macronutriments avec les protéines et les glucides.
Parmi les lipides, on distingue notamment les triglycérides et les phospholipides :

- Les triglycérides (également appelés triacylglycérols ou triacylglycérides ou TAG) sont des glycérides dans lesquels les trois groupements hydroxyles du glycérol sont estérifiés par des acides gras. Ils sont le constituant principal de l'huile végétale et des graisses animales.
  Les triglycérides représentent environ 95 % des lipides alimentaires ingérés par l'Homme. Dans l'organisme, ils sont présents principalement dans les tissus adipeux et constituent la forme principale de stockage de l'énergie.
- Les phospholipides sont des lipides amphiphiles, c'est-à-dire constitués d'une « tête » polaire (hydrophile) et de deux « queues » aliphatiques (hydrophobes).
  Les phospholipides sont des lipides de structure car ils sont des constituants des membranes cellulaires dont ils assurent entre autre la fluidité.

Triglycérides et phospholipides sont composés majoritairement d'acides gras qui sont à la fois apportés par l'alimentation et, pour certains d'entre eux, synthétisés par l'organisme.
La classification biochimique (basée sur le nombre de doubles liaisons contenues dans la molécule d'acide gras) distingue les acides gras saturés (AGS), les acides gras monoinsaturés (AGMI) et les acides gras polyinsaturés (AGPI).
Du point de vue physiologique, on distingue :

- les acides gras indispensables nécessaires au développement et au bon fonctionnement du corps humain, mais que notre corps ne sait pas fabriquer ;
- les acides gras dit « conditionnellement » indispensables, essentiels pour la croissance normale et les fonctions physiologiques des cellules mais qui peuvent être fabriqués à partir de leur précurseur s'il est apporté par l'alimentation. Ils sont donc rigoureusement requis si leur précurseur indispensable est absent.
- les acides gras non indispensables.

L'ensemble des acides gras indispensables et « conditionnellement » indispensables constituent les acides gras essentiels.
Les autres acides gras sont dits non essentiels.
Parmi les acides gras non indispensables, on trouve notamment :

- l'acide eicosapentaénoïque (EPA) de la famille des acides gras oméga 3,
- l'acide oléique, l'acide gras monoinsaturé majoritaire dans notre alimentation, et l'acide palmitoléique,
- les acides gras saturés, tels l'acide laurique, l'acide myristique ou l'acide palmitique.

***Les acides gras polyinsaturés***

Les acides gras polyinsaturés sont classés en fonction de la position de la première double liaison, à partir de la fonction méthyle finale.
Ainsi, dans la nomenclature, pour omega « x » ou « nx », « x » correspond à la position de la première insaturation.
On distingue deux grandes familles d'acides gras essentiels : les acides gras oméga 6 (ou AGPI n-6), dont le précurseur et le représentant majeur est l'acide linoléique (LA) et les acides gras oméga 3 (ou AGPI n-3) dont le précurseur est l'acide alpha-linolénique (ALA).
La majorité des acides gras polyinsaturés d'intérêt biologique appartient à la famille des omega 6 (acide arachidonique ou ARA) ou omega 3 (acide eicosapentaénoïque ou EPA, acide docosahexaénoïque ou DHA).
En outre, dans la nomenclature, on définit également le nombre de carbone constituant la chaîne ; ainsi l'EPA est décrit comme C20:5 et le DHA comme C22:6.
Le « 5 » et « 6 » correspondent ainsi au nombre d'insaturations de la chaîne carbonée présentés respectivement par l'EPA et par le DHA.
Le DHA, de la famille des acides gras oméga 3, est un acide gras que l'organisme sait synthétiser à partir de l'acide alpha-linolénique, ou qui est apporté par la consommation de poissons gras (thon, saumon, hareng...).

Le DHA joue un rôle important dans la structure des membranes et dans le développement et le fonctionnement du cerveau et de la rétine.

Les huiles de poisson sont utilisées principalement comme source d'acides gras de type omega 3, tels le DHA et l'EPA, mais on les trouve également dans les huiles de microalgues à partir desquelles on les extrait soit en mélange, soit séparément, comme c'est le cas par exemple des huiles issues de certaines souches sélectionnées, telles que celles du genre *Schizochytrium,* qui ne contiennent que des traces d'EPA mais de fortes teneurs en DHA.

***Les acides gras saturés***

Parmi les acides gras saturés, l'acide palmitique, également appelé acide hexadécanoïque ou acide cétylique, est l'un des acides gras saturés en C16:0 les plus courants chez les animaux et les plantes.

L'acide palmitique est le premier acide gras produit au cours de la lipogenèse ; à partir de lui, des acides gras plus longs peuvent être produits.

De plus, il est l'acide gras utilisé préférentiellement pour synthétiser de l'ATP. Le bilan énergétique de sa combustion indique 129 ATP. Il constitue ainsi un excellent aliment énergétique.

Industriellement on utilise également l'acide palmitique pour la fabrication aussi bien des margarines que des savons durs.

Dans le domaine des peintures, étant donné qu'il est saturé, l'acide palmitique ne peut pas polymériser et se rigidifier une fois en contact avec l'oxygène de l'air (à la différence de l'acide oléique, linoléique et linolénique). Il reste donc sous sa forme de solide mou et agit (avec l'acide stéarique) comme plastifiant des liants huileux polymérisés. Ainsi, avec l'acide stéarique, il assure l'élasticité nécessaire à la bonne conservation des matières picturales à l'huile, à travers le temps.

***Les acides gras monoinsaturés***

Comme précurseur d'acide gras monoinsaturé, l'acide palmitique conduit à l'acide palmitoléique (16 :1 n-7), naturellement présent en grande quantité dans le fruit ou la pulpe de l'argousier.

Il a par ailleurs été décrit qu'un apport accru d'acide palmitoléique dans l'alimentation pourrait avoir des effets hypocholestérolémiques et hypotriglycéridémiques, réduire les risques d'accident vasculaire cérébral, et améliorer également le métabolisme des cellules musculaires lisses vasculaires.

***Production de lipides, notamment d'acides gras, par les microalgues***

La culture des microalgues du genre *Schizochytrium* est réalisée classiquement dans des fermenteurs (conditions hétérotrophiques : à l'obscurité en présence d'une source carbonée).

Il est à noter que l'exploitation rentable de ces microalgues nécessite généralement la maîtrise des conditions de fermentation.

Pour parvenir à ce résultat, de premiers procédés de fermentation permettant d'obtenir de hautes densités cellulaires (acronyme anglais : HCD pour *High-Cell-Density*) ont été ainsi beaucoup travaillés, de manière à obtenir des rendements et productivités maximales en lipides.

L'objectif de ces cultures HCD était l'obtention de la concentration la plus élevée possible des lipides souhaités dans le laps de temps le plus court.

Cependant, il est vite apparu aux spécialistes du domaine qu'il faut par exemple soumettre les microalgues à un stress nutritionnel qui limite leur croissance lorsqu'on souhaite leur faire produire d'importantes réserves lipidiques.

Il est donc classiquement procédé au découplage croissance / production dans les procédés fermentaires.

Par exemple, pour favoriser l'accumulation d'acides gras polyinsaturés (ici l'acide docosahexaénoïque ou DHA), la demande de brevet WO 01/54510 recommande de dissocier la croissance cellulaire et la production d'acides gras polyinsaturés.

Il est plus particulièrement revendiqué un procédé pour la production de lipides microbiens, comprenant les étapes consistant à :

(a) effectuer une fermentation d'un milieu comprenant des microorganismes, une source de carbone et une source nutritive limitante et en assurant des conditions suffisantes pour maintenir un taux d'oxygène dissous d'au moins environ 4 % de la saturation dans ledit milieu de fermentation pour augmenter la biomasse ;
(b) puis fournir des conditions suffisantes pour maintenir un taux d'oxygène dissous approximativement égal ou inférieur à 1 % de la saturation dans ledit milieu de fermentation et fournir des conditions suffisantes pour permettre auxdits microorganismes de produire lesdits lipides ;
(c) et recueillir lesdits lipides microbiens, dans lequel au moins environ 15 % desdits lipides microbiens sont constitués de lipides polyinsaturés ;

et dans lequel une densité de biomasse d'au moins environ 100 g/l est obtenue au cours de la fermentation.

Chez la microalgue *Schizochytrium sp* souche ATCC 20888, il est ainsi plus particulièrement procédé à une première phase de croissance en présence d'une source carbonée et d'une source azotée mais sans limitation en oxygène, de manière à favoriser l'obtention d'une haute densité cellulaire puis, dans une deuxième phase, arrêter la fourniture d'azote et ralentir progressivement l'apport en oxygène (gestion de la pression en oxygène dissous ou $pO_2$ de 10 %, à 4 %, puis 0,5 %), afin de stresser la microalgue, ralentir sa croissance et enclencher la production des acides gras d'intérêt.

Chez la microalgue *Crypthecodinium cohnii,* la teneur la plus élevée en DHA est obtenue à faible concentration en glucose (de l'ordre de 5 g/l), et ainsi à faible taux de croissance (Jiang and Chen, 2000, Process Biochem., 35(10), 1205-1209).

De ce fait, dans les cas où la formation des produits n'est pas corrélée avec une croissance cellulaire élevée, il est enseigné qu'il est judicieux de maîtriser le taux de croissance cellulaire.

En général, l'homme du métier choisit de contrôler la croissance des microalgues par la maîtrise des conditions de fermentation (Tp, pH...), ou par l'alimentation régulée en composants nutritionnels du milieu de fermentation (conditions semi continues dites « fed-batch »).

S'il choisit de contrôler la croissance des microalgues en hétérotrophie par l'apport en sources carbonées, l'homme du métier choisit généralement d'adapter la source carbonée (glucose pur, acétate, éthanol...) à la microalgue (*C. cohnii, Euglena gracilis*...) en fonction du métabolite produit (par exemple un acide gras polyinsaturé de type DHA).

La température peut également être un paramètre clef. Il a par exemple été rapporté que la synthèse d'acides gras polyinsaturés chez certaines espèces de microalgues, tel que l'EPA par *Chlorella minutissima,* est favorisée à une plus basse température que celle requise pour la croissance optimale de ladite microalgue.

Pour optimiser la production en triglycérides, l'homme du métier est également amené à optimiser le flux carboné vers la production d'huile, en agissant sur l'environnement nutritionnel du milieu de fermentation.

Il est ainsi connu que l'accumulation en huile se produit lors d'un apport carboné suffisant, mais dans des conditions de carence en azote.

Le rapport C/N est donc ici déterminant, et il est admis que les meilleurs résultats sont obtenus en agissant directement sur la teneur en azote, la teneur en glucose n'étant pas limitante.

Pour optimiser la production en huile, il est donc primordial pour l'homme du métier de contrôler le flux carboné en le déviant vers la production d'huile, au détriment de la production des protéines ; le flux carboné est redistribué et s'accumule en substances de réserve lipidiques quand les microalgues sont placées en milieu carencé en azote.

Quoi qu'il en soit, des préparations commerciales de biomasse de microalgues riches en DHA et d'acide palmitique sont disponibles grâce à la mise en oeuvre de conditions de fermentation standard.

Cependant, il demeure un besoin non satisfait de disposer de procédé alternatif de production de biomasses de microalgues de qualité, à haute teneur en DHA et à teneur contrôlée en acide palmitique et/ou acide palmitoléique.

## RESUME DE L'INVENTION

La présente invention est relative à un procédé de production d'une biomasse de microalgues du genre *Thraustochytrium* enrichie en acide docosahexaénoïque (DHA) caractérisé en ce que, pendant la phase de culture en conditions hétérotrophiques, l'apport en oxygène est contrôlé de manière à satisfaire uniquement les besoins en oxygène 1) pour la production d'énergie nécessaire à la maintenance cellulaire, 2) pour la production des lipides de base, et 3) pour la croissance de la biomasse hors acides gras et en ce que l'apport en oxygène nécessaire pour satisfaire uniquement les besoins 1), 2) et 3) est calculé par l'équation 2 suivante

$$qO2Cible = (qm \times \frac{6\times32}{180}) + (qlipidesdebase \times y^{O2}/_{lipides}) + (\mu \times y^{O2}/_{x})$$

dans laquelle

$qO_2Cible$ est la quantité d'oxygène en gramme par gramme de biomasse hors acides gras et heure ;
qm est coefficient de maintenance exprimé en g de glucose par g de biomasse hors acides gras et par heure ;
$q_{lipides\ de\ base}$ est la vitesse d'accumulation des lipides de base exprimée en g de lipides de base par g de biomasse hors acides gras et par heure ;
$y^{O2}/_{lipides}$ est le coefficient de consommation d'oxygène par rapport à la formation de lipides exprimé en g d'oxygène par g de lipides,
$\mu$ est la vitesse de croissance exprimée en g de biomasse hors acides gras formée par g de biomasse hors acides gras et par heure, soit ($h^{-1}$) ;

$y^{O2}/_x$ est le coefficient de consommation d'oxygène par rapport à la formation de biomasse hors acides gras exprimé en g d'oxygène par g de biomasse hors acides gras.

Facultativement, aucune limitation d'un élément nutritif, notamment en source carbonée ou azotée, n'est appliquée pendant le procédé fermentaire.

Facultativement, les phases de croissance et de production de DHA sont concomitantes.

De préférence, les microalgues sont du genre *Schizochytrium sp ou Schizochytrium mangrovei.* De manière plus spécifique, les microalgues peuvent être une souche sélectionnée parmi les souches CNCM I-4469 et CNCM I-4702 déposées à la Collection Nationale de Cultures de Microorganismes de l'Institut Pasteur respectivement le 14 avril 2011 et le 22 novembre 2012.

Facultativement, le procédé peut comprendre en outre la récolte de la biomasse, éventuellement la préparation d'un extrait ou lysat cellulaire à partir de cette biomasse, puis facultativement l'extraction d'une huile brute riche en DHA.

Le procédé selon la présente invention peut être caractérisé en ce que la biomasse obtenue comprend

- au minimum 40 % de DHA en poids d'acides gras totaux ; et/ou
- au maximum 40 % d'acide palmitique en poids d'acides gras totaux ; et/ou
- au minimum 25 % d'acides gras en sec poids de biomasse.

## DESCRIPTION DETAILLEE DE L'INVENTION

Dans le cadre de l'invention, la société Demanderesse a choisi d'explorer une voie originale d'optimisation de la production en DHA en proposant une solution alternative à celles classiquement envisagées par l'homme du métier.

La société Demanderesse a ainsi trouvé, ce qui va à l'encontre des préjugés techniques en la matière, qu'il est possible de produire par fermentation des biomasses de microalgues riches en lipides (plus de 45 % en poids sec de biomasse) dont les acides gras prépondérants sont l'acide docosahexaénoïque (DHA), tout en modulant ou contrôlant la quantité d'acide palmitique et d'acide palmitoléique :

- sans qu'il ne soit nécessaire de découpler la phase de croissance de la microalgue et la phase de production des lipides - au contraire, la production de lipides est concomitante à la croissance cellulaire et est donc réalisée en une seule phase,
- sans qu'il ne soit indispensable, comme décrit dans l'état de l'art, d'induire une limitation en azote ou en un quelconque autre élément nutritif, et
- sans qu'il ne soit non plus indispensable de piloter la fermentation par la $pO_2$.

La société Demanderesse a ainsi trouvé que l'on peut contrôler la composition en lipides de la biomasse, et notamment les proportions de DHA et d'acide palmitique et palmitoléique grâce à un contrôle de l'oxygénation du milieu de fermentation.

En effet, la société Demanderesse a compris que l'oxygène est utilisé par les microalgues selon les priorités suivantes :

- production d'énergie pour la maintenance,
- production d'une palette d'acides gras, appelés lipides de base, dont le DHA est majoritaire,
- croissance de la biomasse hors acides gras, et
- production d'acide palmitique avec le surplus d'oxygène.

En d'autres termes, le procédé conforme à l'invention consiste à satisfaire les besoins en oxygène correspondants aux trois premiers points et ainsi d'apporter la quantité d'oxygène optimale pour obtenir une biomasse riche en DHA (qu'elle soit produite par *Schizochytrium sp ou S. mangrovei*).

Ensuite, comme il sera démontré dans la partie expérimentale ci-après, un apport d'oxygène supplémentaire peut être réalisé et conduit à une surproduction rapide d'acide palmitique (bien illustré avec *S. mangrovei*), avec ou sans acide palmitoléique (bien illustré avec *Schizochytrium sp*).

La société Demanderesse a commencé ses travaux sur la base de la souche *Schizochytrium sp* ATCC 20888.

En suivant les enseignements de l'état de l'art, notamment ceux de la demande de brevet WO 01/54510, la société Demanderesse a tout d'abord trouvé que, contrairement à ce qui est divulgué, une régulation constante de la $pO_2$ à 0 % ou à plus de 10 % pendant toute la durée de la fermentation permettait de produire plus de 35 % d'AGPI dans les acides gras (plus de 25 % de DHA sur acides gras totaux), tout comme avec une conduite raisonnée avec une réduction de la $pO_2$ en cascade (10 %, puis 4 %, puis 0,5 % de saturation).

En revanche, cette dernière conduite (selon la demande WO 01/54510) appliquée à la souche CNCM I-4702 aboutit au final à la production d'acides gras dont l'acide palmitique est très majoritaire à plus de 60%, alors que le DHA n'atteint pas 20% ; cette conduite aboutit aux mêmes résultats que ceux obtenus pour cette souche avec une conduite à $pO_2$ à 10 % constant.

En outre, d'un point de vue technique, la mesure de la $pO_2$ pose de grandes difficultés lorsqu'il s'agit de transposer le protocole du laboratoire à l'échelle industrielle (en d'autres termes, passer de l'échelle des fermenteurs de 2 à 20 l à l'échelle de réacteurs de 1 à 200 $m^3$).

En effet, la $pO_2$ est définie comme la concentration relative en oxygène dissous dans le moût de fermentation à saturation. Par exemple, si de l'eau est mise en aération sous air, à température ambiante et sous pression atmosphérique, suffisamment longtemps, on considère que la $pO_2$ est égale à 100 % (ce qui correspond à l'état de l'oxygénation du fermenteur à t = 0). Or, lors du calibrage d'une sonde $pO_2$ dans un fermenteur, la teneur en oxygène dissous est influencée par la concentration en sels résiduels et par la température de fermentation.

Par ailleurs, il est classiquement admis que, pour un fermenteur de laboratoire, la $pO_2$ est peu influencée par la pression générée par la hauteur du moût de fermentation et par les effets de mélange. Cependant, lors des industrialisations sur des fermenteurs de moyenne (de l'ordre du $m^3$) à grande capacité (de l'ordre de centaines de $m^3$), la hauteur du moût de fermentation va au contraire :

- avoir une influence sur la pression en oxygène dissous ; et
- provoquer des phénomènes complexes dans le fermenteur « non parfaitement agité ».

En ce sens, la valeur de $pO_2$ établie à l'échelle du laboratoire n'est donc pas extrapolable à l'échelle industrielle.

De plus, les préconisations du brevet WO 01/54510 décrit pour *Schizochytrium sp* ne semblent pas généralisables à toutes les souches du genre *Thraustochytrium.* En d'autres termes, dans la présente invention, il n'est pas procédé ici à une diminution de l'apport en oxygène de manière à « stresser » la microalgue afin de lui faire produire ses lipides de réserves (en l'occurrence le DHA), mais à contrôler l'apport en oxygène à hauteur des besoins exprimés par ladite microalgue pour moduler l'orientation de sa production de lipides et, ce sans se référer à la $pO_2$.

Par ailleurs, le choix de la conduite de fermentation en contrôlant l'apport d'oxygène a conduit la société Demanderesse à obtenir, sans qu'il ne soit utile de limiter l'apport en certaines substances nutritives ni de découpler phase de croissance et de production, de remarquables résultats :

- une augmentation de la teneur globale en lipides d'intérêt, et notamment du DHA, et
- une réduction des odeurs (la réduction de l'odeur particulièrement intense des biomasses récoltées de la CNCM I-4469 est remarquable.

***Choix des microorganismes***

Les souches à utiliser dans les méthodes de la présente invention sont du genre *Thraustochytrium,* plus particulièrement *Schizochytrium sp ou Schizochytrium mangrovei.* De telles souches sont connues de l'homme du métier. Par exemple, on peut citer la souche *Schizochytrium sp* ATCC No 20888, décrite et étudiée dans la demande WO 01/54510.

La société Demanderesse a identifié au cours de ses recherches plusieurs souches de microalgues productrices de DHA de grand intérêt. Notamment, la société Demanderesse est tout particulièrement intéressée par deux souches qu'elle a identifiées.

La première souche est une souche de *Schizochytrium sp.,* déposée en France le 14 avril 2011 auprès de la Collection Nationale de Cultures de Microorganismes de l'Institut Pasteur (CNCM) sous le numéro I-4469 mais également en Chine auprès du CHINA CENTER FOR TYPE CULTURE COLLECTION (CCTCC) de l'université de Wuhan, Wuhan 430072, P.R. China sous le numéro M 209118. Cette souche produit principalement du DHA et en moindre mesure de l'acide palmitique et de l'acide palmitoléïque. Elle a été caractérisée par séquençage partiel du gène codant pour l'ARN 18S (SEQ ID No 1):

```
1   GAGGGTTTTA CATTGCTCTC aTTCCaATAG CAaGACGCGA AGCGCCCCGC ATTGATATTT
61  CTCGTCACTA CCTCGTGGAG TCCACATTGG GTAATTTACG CGCCTGCTGC CTTCCTTGGA
121 TGTGGTAGCC GTCTCTCAGG CTCCCTCTCC GGAGTCGAGC CCTAACTCCC CGTCACCCGT
181 TATAGTCACC GTAGGCCAAT ACCCTACCGT CGACAACTGA TGGGGCAGAA ACTCAAACGA
241 TTCATCGCTC CGAAAAGCGA TCTGCTCAAT TATCATGACT CACCAAGAGA GTTGGCTTAG
301 ACCTAATAAG TGCGGCCCTC CCCGAAAGTC GGGCCCGTAC AGCACGTATT AATTCCAGAA
361 TTACTGCAGG TATCCGTATA AAGGAACTAC CGAAGGGATT ATAACTGATA TAATGAGCCG
421 TTCGCAGTTT CACAGTATAA TTCGCTTATA CTTACACATG CATGGCTTAG TCTTTGAGA
```

ce qui a permis de l'identifier comme étant une souche du type *Schizochytrium sp.* Cette souche sera désignée « CNCM I-4469» ultérieurement dans la présente demande.

Par ailleurs, la deuxième souche est une souche de *Schizochytrium mangrovei.* Elle produit du DHA et d'acide palmitique en proportion relativement égale. Elle a été déposée par la société Demanderesse en France le 22 novembre 2012 auprès de la Collection Nationale de Cultures de Microorganismes de l'Institut Pasteur (CNCM) sous le numéro CNCM I-4702. Elle a été caractérisée par séquençage des gènes codant pour l'ARNr 18 S (SEQ ID No 2):

```
  1 GGTTTTACAT TGCTCTCATT CCGATAGCAA AACGCATACA CGCTTCGCAT CGATATTTCT
 61 CGTCCTACCT CGTGGAGTCC ACAGTGGGTA ATTTACGCGC CTGCTGCTAT CCTTGGATAT
121 GGTAGCCGTC TCTCAGGCTC CCTCTCCGGA GTCGAGCCCT AACTCTCCGT CACCCGTTAT
181 AGTCACCGTA GTCCAATACA CTACCGTCGA CAACTGATGG GGCAGAAACT CAAACGATTC
241 ATCGACCAAA AWAGTCAATC TGCTCAATTA TCATGATTCA CCAATAAAAT CGGCTTCAAT
301 CTAATAAGTG CAGCCCCATA CAGGGCTCTT ACAGCATGTA TTATTTCCAG AATTACTGCA
361 GGTATCCATA TAAAAGAAAC TACCGAAGAA ATTATTACTG ATATAATGAG CCGTTCGCAG
421 TCTCACAGTA CAATCGCTTA TACTTACACA GCAG
```

ce qui a permis de l'identifier comme étant une souche du type *Schizochytrium mangrovei.* Cette souche sera désignée « CNCM I-4702» ultérieurement dans la présente demande.

***Détermination et utilisation de la quantité d'oxygène nécessaire, en particulier $qO_2$***

Le flux d'oxygène global introduit dans le milieu de culture est modulable et dépend essentiellement :

- du débit de l'air introduit,
- de la vitesse d'agitation qui favorise la dissolution de l'oxygène dans le milieu.

Ce flux est nommé OTR (acronyme anglais pour Oxygen Transfer Rate ou Taux de transfert d'oxygène).

La consommation d'oxygène par la culture est nommée OUR (acronyme anglais pour Oxygen Uptake Rate ou Taux de consommation d'oxygène).

Lorsque la biomasse croît dans le fermenteur, sa consommation globale en oxygène augmente jusqu'à absorber tout l'oxygène transféré, l'OUR est alors considérée comme égale à l'OTR.

L'OTR et l'OUR sont des données pour l'ensemble du milieu de culture. Ainsi, l'OUR pour un volume de culture peut être rapporté à la quantité de cellules qui occupent ce volume.

Ainsi, la consommation cellulaire est nommée $qO_2$ et représente la quantité d'$O_2$ en gramme absorbée par gramme de cellule et par heure. Cette consommation est alors calculée pour la masse de cellules en tant que telle, c'est-à-dire sans la masse des acides gras, puisque les réserves lipidiques n'ont pas de rôle actif dans l'absorption de l'oxygène.

Le procédé de l'invention permet, par un apport contrôlé en oxygène au milieu de culture, d'agir sur la production des lipides et de l'optimiser en fonction des conditions de croissance des microalgues (taille de l'inoculum, âge de la culture...).

La consommation d'$O_2$ globale correspond à l'addition des différentes utilisations de l'oxygène par la cellule.

La société Demanderesse distingue quatre voies de consommation de l'oxygène, qui correspondent aux besoins élémentaires de la cellule, besoins satisfaits successivement avec un ordre de priorité à hauteur de l'apport en oxygène.

Ces besoins sont les suivants, par ordre de priorité :

1. La maintenance cellulaire.
2. La production de lipides de base.
3. La formation de la biomasse hors acide gras.
4. Une accumulation de lipides liés à un débordement métabolique.

La société Demanderesse a trouvé que le $qO_2$ se détermine alors par la formule suivante :

Equation (1)

$$qO2 = \left(qm \times \frac{6\times 32}{180}\right) + \left(qlipidesdebase \times y^{02}/_{lipides}\right) + \left(\mu \times\ y^{O2}/_{x}\right) + \left(qlipidesdedébordement \times y^{02}/_{lipides}\right)$$

(1) (2) (3) (4)

Les termes « qm », « $q_{lipides\ de\ base}$», « y $^{O}{}_{2/lipides}$ », « $\mu$ », « y $^{O}{}_{2/x}$ » et « $q_{lipides\ de\ débordement}$ » de l'équation doivent être compris comme suit.

***La maintenance cellulaire. Terme (1) de l'Equation***

La maintenance cellulaire correspond à l'énergie dont a besoin la cellule pour son entretien, indépendamment de toute production de lipides ou de croissance de biomasse.

Le « qm » est la quantité de substrat carboné (le glucose généralement) utilisée pour produire cette énergie ; il est exprimé en g de glucose par g de biomasse hors acides gras et par heure. Ce terme est classiquement désigné dans le domaine comme l'énergie de maintien ou coefficient de maintenance.

Le besoin en oxygène associé est proportionnel. Il faut en effet 6 molécules d'$O_2$ (Masse Molaire : 32 g) pour transformer une molécule de glucose (Masse Molaire : 180 g). D'où le terme « $qm \times \frac{6\times 32}{180}$ » dans l'équation (1).

***La production de lipides de base. Terme (2) de l'Equation***

La société Demanderesse a constaté que l'accumulation de lipides est concomitante à la croissance et n'est pas uniquement un phénomène apparaissant à l'arrêt de cette dernière. Une accumulation de lipides est précurseur de la croissance, ne serait-ce que pour la formation des membranes cellulaires.

La composition de ces lipides de base est différente en fonction de la souche et comprend entre autres de l'acide palmitique ou de l'acide palmitoléïque comme précurseurs de la biosynthèse du DHA, mais le DHA est toujours le constituant majoritaire, dont la quantification est illustrée par l'exemple 2.

La vitesse d'accumulation de ces lipides correspond à un flux nommé « $q_{lipides\ de\ base}$ » dans l'équation (1).

Pour les souches CNCM I-4469 *Schizochytrium sp* et CNCM I-4702 *Schizochytrium mangrovei,* cette vitesse est exemplifiée ci-après.

Pour obtenir le besoin en oxygène associé, on multiplie ce flux ou vitesse par le besoin en oxygène pour former des lipides (également appelé coefficient de consommation d'oxygène par rapport à la formation de lipides), exprimé par le terme « y $^{O}{}_{2}/_{Lipides}$ » dans l'équation (1).

Ainsi, l'apport en oxygène servira en second lieu la production de lipides de base.

***La formation de la biomasse hors acide gras. Terme (3) de l'Equation***

La biomasse totale comprend les réserves lipidiques.

Ces dernières (constitués par les acides gras) sont déterminées par dosage et sont soustraites de la biomasse totale.

La formation de la biomasse hors acides gras (hors « A.G. ») correspond à la croissance de la biomasse riche en protéines, et donc réellement active.

La vitesse de croissance est symbolisée par « $\mu$ » dans l'équation (1) qui représente la biomasse (hors A.G.) en g formée par g de biomasse (hors A.G.) et par heure soit ($h^{-1}$).

Pour obtenir le besoin en oxygène associé, on multiplie cette vitesse de croissance ($\mu$) par le besoin en oxygène pour former la biomasse hors A.G., exprimé par le terme : « y $^{O2}/_{X}$ » dans l'équation (1).
Ainsi, l'apport en oxygène servira en troisième lieu la production de biomasse hors acides gras.

***Calcul de la concentration en biomasse hors acide gras***

La croissance est continue car, conformément au procédé de l'invention, il n'y a aucune limitation nutritionnelle. Cependant, il est observé un ralentissement de la vitesse de croissance indépendant de l'épuisement du milieu.
La concentration en biomasse hors A.G. varie selon la concentration en biomasse initiale et augmente selon son taux de croissance.
Pour prédire par le calcul la concentration en biomasse à chaque instant et estimer la valeur du taux de croissance, la société Demanderesse recommande d'utiliser l'équation (3) suivante.

Equation (3)

$$\mu = \mu^{\max}\left(1 - \frac{X}{X^{\max}}\right)$$

Cette équation illustre la réduction observée et inexpliquée du taux de croissance.
« $\mu$ » représente le taux de croissance (exprimé en $h^{-1}$) et X la concentration cellulaire hors A.G. (exprimée en g/L).
Les paramètres sont différents pour les deux souches préférées et repris dans le tableau I.

Tableau I

| Souche | $\mu$ max | X max (hors A.G.) |
|---|---|---|
| CNCM I-4469 | 0,08 $h^{-1}$ | 45 g/L |
| CNCM I-4702 | 0,17 $h^{-1}$ | 40 g/L |

Ce phénomène réduit aussi le $qO_2$, car le taux de croissance ($\mu$) contribue à la consommation d'oxygène (Equation 1).
La concentration en biomasse est calculée à partir de la quantité de biomasse introduite et connue (Inoculum) et de son taux de croissance qui évolue.

***L'accumulation de lipides liés à un débordement métabolique. Terme (4) de l'Equation***

Il a été déterminé par la société Demanderesse que ce dernier flux n'existe qu'à partir du moment où les besoins précédemment cités ont été satisfaits et que l'on apporte l'oxygène nécessaire.
Cet apport d'oxygène, exprimé dans l'équation (1) par le terme « $q_{\text{lipides de débordement}}$ » x « y $^{O_2}/_{\text{lipides}}$ » provoque une augmentation de la vitesse de consommation du glucose qui sera transformé en un flux d'acide gras. Cet apport d'oxygène supplémentaire pourra être modulé de manière à contrôler la production des lipides de débordement.
Cet accélération de la production d'acide gras, telle qu'illustrée dans l'exemple 1, provoque un débordement métabolique et aboutit à une accumulation d'acide palmitique pour *Thraustochytrium* et d'acides palmitique et palmitoléique pour *Schizochytrium sp.* Sans être liée par cette théorie, la société Demanderesse considère que ce débordement est lié au fait que les enzymes en aval de la voie de métabolisation continuent à agir au même rythme alors que les enzymes du début de la voie accélèrent leur activité.

***Calcul du Besoin en $O_2$ pour former les A.G. et la biomasse hors A.G.***

Les rendements de conversion sur $O_2$ permettent de connaitre les consommations d'$O_2$ en fonction des productions de biomasse hors A.G ou de lipides.
Les rendements de conversion sont des paramètres bien connus de l'homme du métier qui peut donc les déterminer par des expériences de routine.
Ces valeurs sont indiquées tableau II et elles sont propres aux deux souches préférées.

Tableau II

| | | | |
|---|---|---|---|
| Besoin en $O_2$ pour la production de biomasse hors A.G. | (g/g) | 0,80 | $Y^{O2}/_{x}$ |
| Besoin en $O_2$ pour la production des lipides | (g/g) | 0,17 | $Y^{O2}/_{lipides}$ |

***Obtention d'une biomasse riche en DHA - Calcul du $qO_2$ cible***

Pour augmenter la teneur en DHA, la société Demanderesse a établi qu'il était approprié de contrôler l'apport en oxygène pour ne satisfaire que les 3 premières utilisations de l'oxygène (maintenance, production de lipide de base, et croissance de la biomasse hors A.G.). Cet apport peut être défini par la détermination du $qO_2$ cible, qui correspond à la quantité d'$O_2$ nécessaire en gramme par gramme de cellule et par heure.

Le $qO_2$ cible est alors défini par l'équation (2), qui ne comprend que les trois premières composantes de l'équation (1)

Equation 2 :

$$qO2Cible = \left(qm \times \frac{6\times 32}{180}\right) + \left(qlipidesdebase \times y^{O2}/_{lipides}\right) + \left(\mu \times y^{O2}/_{x}\right)$$

(1) (2) (3)

Ensuite, cette vitesse de consommation d'$O_2$ cellulaire est transposée pour le fermenteur en la multipliant par la concentration en biomasse hors A.G., qui peut soit être mesurée soit prédite par calcul pour chaque instant. Cette vitesse correspond à l'OUR.

Etant donné que l'on se place dans des conditions où l'OTR correspond à l'OUR, ce sera donc cet OTR qui sera appliqué pour chaque instant pendant le procédé fermentaire.

La société Demanderesse a trouvé que c'est par le respect de ce contrôle de l'oxygénation cible que l'on obtient une biomasse riche en DHA. Une oxygénation supérieure à la cible aboutit à produire plus d'acide palmitique et/ou d'acide palmitoléique et ainsi à diluer le DHA. Une oxygénation inférieure au niveau d'oxygénation cible limite la quantité de biomasse produite.

Le $qO_2$ cible variant avec l'évolution de la culture, il est fait référence dans les exemples suivants à l'OUR maximal constaté. Les exemples 1 et 2 illustrent l'effet du contrôle de l'oxygénation sur les souches CNCM I-4469 *Schizochytrium sp* et CNCM I-4702 *Schizochytrium mangrovei* et la méthode pour obtenir les vitesses de production spécifiques. L'exemple 3 illustre, pour la souche CNCM I-4702 *Schizochytrium mangrovei,* l'effet d'une conduite dans laquelle la variation de l'oxygénation permet de modifier les proportions d'acide palmitique et de DHA de la biomasse.

Dans un mode de réalisation alternatif, l'apport en oxygène nécessaire pour satisfaire les trois premiers besoins, à savoir les besoins (1), (2) et (3), peut également être déterminé de manière empirique. Comme l'illustre l'exemple 3, l'homme du métier peut réaliser différents procédés de fermentation dans lesquelles une gamme d'OTR est mise en oeuvre et les quantités de lipides et de biomasse sont mesurées. Sur la base de ces résultats, l'homme du métier peut définir l'OTR cible qui permet de satisfaire les trois premiers besoins.

Le procédé fermentaire selon la présente invention permet d'obtenir une biomasse riche en acides gras. Notamment, la biomasse comprend au minimum 25 % d'acides gras en sec poids de biomasse, de préférence au minimum 30 %. Le taux d'acides gras peut dépendre de la souche mise en oeuvre et peut atteindre un minimum de 40 % en poids sec de biomasse pour la souche I-4702.

Par ailleurs, et de manière tout à fait intéressant, cette biomasse riche en lipides présente un taux élevé de DHA. Notamment, le procédé fermentaire selon la présente invention permet d'obtenir une biomasse comprenant au minimum 40 % en poids de DHA par rapport aux acides gras totaux.

Enfin, le procédé fermentaire selon la présente invention permet d'obtenir une biomasse présentant une teneur réduite en acide palmitique. Ainsi, la biomasse comprenant au maximum 40 % en poids de DHA par rapport aux acides gras totaux. Le taux d'acide palmitique peut dépendre de la souche mise en oeuvre et peut atteindre un maximum de 10 % en poids par rapport aux acides gras totaux pour la souche I-4469.

Par ailleurs, la présente invention considère également des procédés fermentaires dans lesquels l'apport en oxygène est supérieur à celui nécessaire pour satisfaire les trois premiers besoins, à savoir les besoins (1), (2) et (3). Notamment, cet apport sera contrôlé de manière à obtenir les proportions relatives en DHA et acides palmitique et/ou palmitoléïque souhaitées, tout en optimisant la quantité de biomasse produite.

Par ailleurs, les procédés fermentaires selon la présente invention sont mis en oeuvre dans des conditions de culture hétérotrophiques. Ces conditions adaptées aux microalgues considérées ainsi que les milieux de culture sont bien connus de l'homme du métier. La source carbonée nécessaire à la croissance de la microalgue est préférentiellement du glucose. La source d'azote peut être des extraits de levure, de l'urée, du glutamate de sodium, du sulfate d'ammonium, de l'ammoniaque en régulation de pH, pris seuls ou en combinaison. Généralement, l'étape de culture comprend une étape de préculture, pour revivifier la souche, puis une étape de culture ou de fermentation proprement dite. Cette dernière étape correspond à l'étape de production des lipides d'intérêt, en particulier de DHA.

Outre la biomasse, la présente invention concerne également un extrait ou lysat cellulaire préparé à partir de cette biomasse. En particulier, cet extrait ou lysat est préparé à partir de la biomasse récupérée après fermentation. Cet extrait ou lysat riche en DHA, et facultativement en acides palmitique et/ou palmitoléïque. La rupture des cellules pour l'extraction du contenu lipidique peut être effectuée par différentes voies parmi lesquelles les voies mécanique, chimique, enzymatique.

Par la suite, une huile peut être extraite du lysat cellulaire, par exemple à l'aide hexane/éthanol en plusieurs extractions successives. La fraction hexanique est ensuite séparée puis l'hexane est évaporé pour isoler l'huile brute.

Ainsi, la méthode de production de lipides d'intérêt, de préférence DHA, et facultativement d'acides palmitique et/ou palmitoléïque, comprend le procédé fermentaire selon la présente invention, la récolte de la biomasse, la préparation d'un extrait ou lysat cellulaire et extraction d'une huile brute comprenant les lipides d'intérêt, de préférence DHA, et facultativement d'acides palmitique et/ou palmitoléïque.

## EXEMPLES

<u>Exemple 1</u> : **Conditions de cultures des souches CNCM I-4469 et CNCM I-4702 et détermination des vitesses spécifiques de production en excès d'oxygénation**

<u>*Conditions de cultures*</u>

Le protocole comprend une préculture en Erlens pour un ensemencement du fermenteur à 0,1 g de biomasse/L pour la souche CNCM I-4469 et au minimum 5g de biomasse/L pour la souche CNCM I-4702.

***Préculture***

La préculture (100 ml de milieu) en Erlens de 500 ml à chicanes dure 24 h à une température de 28°C.

L'ensemble des composants du milieu est stérilisé par filtration et introduit dans un Erlen préalablement stérilisé à l'autoclave après ajout d'une goutte d'anti-mousse Clearol FBA 3107.

Tableau III

| Milieu de pré culture % (g/g) | |
|---|---|
| Glucose anhydre | 3 |
| Extrait de levure | 0,4 |
| Glutamate de sodium mono-sodique | 6,42 |
| NaCl | 1,25 |
| $MgSO_4$ 7($H_2O$) | 0,4 |
| KCl | 0,05 |
| $CaCl_2$2 ($H_2O$) | 0,01 |
| $NaHCO_3$ | 0,05 |
| $KH_2PO_4$ | 0,4 |
| Vitamines solution mère B1, B6, B12 | 0,1 |
| Oligo-éléments solution mère | 0,8 |

***Culture***

Le milieu est stérilisé en 3 parties.

Le glucose est stérilisé avec le $KH_2PO_4$ en Erlen pour un ajout juste avant $T_0$.

Le reste des sels est stérilisé en fermenteur avec 0,05 ml /L de Clearol FBA 3107. Les Oligo-éléments et vitamines sont stérilisés par filtration.

Le volume à $T_0$ représente 75 % du volume final. Le pH est ajusté à $T_0$ par de l'ammoniaque puis il est régulé à 6 toujours à l'ammoniaque.

Tableau IV

| Milieu de culture % (P/P) | |
|---|---|
| $KH_2PO_4$ | 0,80 |
| $(NH_4)_2SO_4$ | 0,33 |
| $Na_2SO_4$ | 0,67 |
| NaCl | 0,27 |
| Ca $Cl_2$ 2 ($H_2O$) | 0,03 |
| Mg $SO_4$ 7($H_2O$) | 1,00 |
| Glucose anhydre | 6,00 |
| Vitamines solution mère B1, B6, B12 | 0,20 |
| Oligo-éléments solution mère | 0,27 |

Un Fed batch de glucose (concentration : 500 g/L de Fed) est apporté en continu à partir de $T_0$ à un rythme constant (à adapter d'après calculs) pour ne pas être à une concentration inférieure à 20 g/L et 5g/L au final.

La culture est conduite à la température de 28 °C et dure de 65 à 85 heures.

L'utilisation de liqueur d'eau de trempe du maïs (acronyme « CSL ») ou d'extraits de levures (acronyme « EL ») comme source d'azote est possible, permettant d'obtenir des résultats légèrement supérieurs en DHA.

***Solutions Mères***

Tableau V

| Oligo-éléments | g/L |
|---|---|
| $MnCl_2$ $2H_2O$ | 8,60 |
| $CoCl_2$ $6H_2O$ | 0,2 |
| $NiSO_4$ $6H_2O$ | 7,50 |
| $Na_2MoO_4$ $2H_2O$ | 0,15 |
| $ZnSO_4$ $7H_2O$ | 5,70 |
| Cu $So_4$ $5h_2O$ | 6,50 |
| $FeSO_4$ 7 $H_2O$ | 32,00 |
| Acétate de Zinc | 0,01 |
| EDTA | Mis à pH < 3 |

Tableau VI

| Vitamines | g/L |
|---|---|
| B1 | 45 |
| B6 | 45 |
| B12 | 0,25 |

*Détermination des vitesses de production spécifiques*

Pour évaluer les vitesses de production spécifiques, une première culture a été réalisée à $pO_2$ 10%, ce qui

signifie que l'on s'assure qu'il reste toujours de l'oxygène dissous dans le milieu. Cette culture a été menée sans limitation en sources nutritives.

Cette méthode permet de mettre en évidence les caractéristiques des souches à tester, en excès d'oxygène (Figure 1).

Dans le tableau VII, les résultats présentés sont ceux obtenus à T65 de la culture des biomasses des deux souches CNCM I-4469 et CNCM I-4702.

Tableau VII : *Biomasses et compositions en acides gras*

| | CNCM I-4469 | CNCM I-4702 |
|---|---|---|
| Biomasse (g/L) | 51,2 | 79 |
| Teneur en A-G / Biomasse totale (g/g) | 0,38 | 0,57 |
| DHA / A.G. (g/g) | 0,34 | 0,19 |
| acide palmitique/ A.G (g/g) | 0,28 | 0,67 |
| Autres acides gras /A.G. (g/g) | 0,38 | 0,14 |
| A.G. signifiant Acide Gras | | |

Les différents acides gras autres que DHA et acides palmitiques (notamment acide palmitoléique) sont regroupés sous l'appellation « autres acides gras » pour mettre en évidence l'effet sur le DHA et l'acide palmitique, mais rejoignent les lipides de base avec le DHA dans le calcul du $qO_2$ cible.

Le calcul des vitesses moyennes est réalisé globalement.

La biomasse finale est analysée par Chromatographie en Phase Gaz (CPG). On connaît alors la concentration en biomasse totale, la teneur de chacun des acides gras et la teneur globale des acides gras.

La biomasse hors acide gras, appelée également biomasse active, est calculée par retranchement de ces acides gras à la biomasse totale.

La quantité de chacun des acides gras produits est ensuite divisée par la moyenne de la biomasse hors A.G. présente et par le temps. Le résultat obtenu est une vitesse spécifique globale de production par unité de temps et par g de biomasse hors A.G.

Ces vitesses sont présentées dans le tableau VII suivant.

Tableau VIII : *Calcul des vitesses*

| | CNCM I-4469 | CNCM I-4702 |
|---|---|---|
| μ biomasse active ($h^{-1}$) | 0,05 | 0,055 |
| q Lipides (g/g/h) | 0,034 | 0,074 |
| q(DHA) (g/g/h) | 0,011 | 0,014 |
| q(palm) (g/g/h) | 0,009 | 0,050 |
| q(autres A.G.) (g/g /h) | 0,013 | 0,010 |

<u>Exemple 2</u> : **Cultures des souches CNCM I-4469 et CNCM I-4702 avec un apport d'oxygène contrôlé**

Les mêmes conditions de cultures ont été mise en oeuvre mais l'apport en oxygène a été contrôlé.

La méthode consiste à utiliser la moitié de la valeur du transfert observé (OUR) lors de la culture à 10 % (Ici ; 50 mmoles /Uh cf. graphique 1 soit 25 mmoles /Uh) (Figure 2).

Par ailleurs, pour un fermenteur de 20 l, pour respecter le bon fonctionnement du fermenteur, quels que soient les besoins en oxygénation, on fixe à $t_0$ une agitation minimum, de l'ordre de 150 rpm, pendant les 10 à 15 premières heures de culture.

Dans le tableau IX, les résultats présentés sont ceux obtenus à T65 de la culture des biomasses des deux souches CNCM I-4469 et CNCM I-4702 dans des conditions d'apport en $O_2$ contrôlée.

Tableau IX : *Biomasses et compositions en acides gras*

| | CNCM I-4469 | CNCM I-4702 |
|---|---|---|
| Biomasse (g/L) | 34 | 59 |
| Teneur en A-G / Biomasse totale (g/g) | 0,29 | 0,45 |
| DHA / A.G. (g/g) | 0,52 | 0,44 |
| acide palmitique/ A.G (g/g) | 0,04 | 0,39 |
| Autres acides gras /A.G. (g/g) | 0,44 | 0,17 |

Le calcul des vitesses moyennes est réalisé globalement comme indiqué dans l'exemple 1. Les résultats sont donnés dans le Tableau X.

Tableau X : *Calcul des vitesses*

| | CNCM I-4469 | CNCM I-4702 |
|---|---|---|
| $\mu$ biomasse active ($h^{-1}$) | 0,05 | 0,055 |
| q Lipides (g/g/h) | 0,021 | 0,045 |
| q(DHA) (g/g/h) | 0,011 | 0,02 |
| q(palm) (g/g/h) | 0,001 | 0,018 |
| q(autres A.G.) (g/g /h) | 0,009 | 0,007 |

En comparant les vitesses spécifiques globales de production en conditions d'excès en oxygène (valeurs du tableau VIII) et en conditions contrôlées en oxygène, on constate que :

- l'apport d'oxygène a permis de diminuer la vitesse de production d'acide palmitique,
- la vitesse de production d'acide palmitique est diminuée d'un facteur 9 pour la souche CNCM I-4469 et par 3 pour la souche CNCM I-4702.

Ce résultat est obtenu alors que les vitesses de production de biomasse ou de DHA restent inchangées.
Ainsi, on prévient la production de lipides dans le contexte d'un débordement métabolique.
Les vitesses évaluées par cette méthode servent de base au calcul du $qO_2$ Cible.

*Calcul du $qO_2$ avec les équations 1 et 2*

Les vitesses de production des lipides du tableau X représentent ici le flux constant des lipides de base ($q_{lipidesdebase}$ dans les équations 1 et 2), dont le DHA est le constituant majoritaire mais qui comprend aussi un peu d'acide palmitique.
Le flux de débordement métabolique correspond au flux supplémentaire, entre les vitesses du tableau VIII et celle du Tableau X.
Le qm est négligeable dans les conditions de culture non stressantes ; on retient une valeur de 0,006 g/g/h.
Le taux de croissance se réduit avec l'augmentation de la concentration en biomasse ; le taux de croissance max peut être maintenu jusque 4 g/L de biomasse hors lipides pour *CNCM I-4702 et 7 g/L* pour CNCM I-4469 ensemencé à 5 g/L.
On peut transposer le qO2 (consommation au niveau cellulaire) à l'OTR (apport correspondant au niveau du fermenteur) par la prise en compte de la concentration en biomasse.
Pour la souche CNCM I-4469 en se plaçant à $\mu^{max}$

EQ 2 :

$$qO_{2Ciblex}=0.006 *1,07 +(0,011+0,001+0.009)* 0,17 + 0,08 *0,8 =0.106 \text{ g/g/h}$$

ce qui correspond à :

$$OTR_{Cible} = 0{,}106 * 7 = 0{,}74 \text{ g d'}O_2 \text{ par L de culture et par heure}$$

ou

$$OTR_{Cible} = 0{,}106 * 7 * 1000 / 32 = \mathbf{23 \text{ mmoles d'}O_2 \text{ /L de culture et par /h}}$$

*Pour la souche CNCM I-4702 en se plaçant à* $\mu^{max}$

EQ 2 :

$$qO_{2Cible} = 0.006 *1{,}07 + (0{,}02+0{,}018+0{,}07) * 0{,}17 + 0{,}17 * 0{,}8 = 0{,}218 \text{ g/g/h}$$

ce qui correspond à :

$$OTR_{Cible} = 0.218 *4=0{,}87 \text{ (g de } O_2 \text{ par L de culture et par heure)}$$

$$OTR_{Cible} = 0.218 *4 *1000 / 32 = \mathbf{27{,}4 \text{ mmoles d'}O_2 \text{ /L de culture et par /h}}$$

L'utilisation de ces équations renseignées par les données du tableau X permet de simuler la culture, et déterminer $OTR_{cible}$ à appliquer.

Ce dernier se réduit avec la baisse du taux de croissance, mais ceci est partiellement compensé par l'augmentation de la concentration en biomasse.

L'$OTR_{cible}$ reste donc voisin de celui exprimé ci-dessus jusqu'à atteindre un taux de croissance de la biomasse hors lipides nul.

**Exemple 3 : Conduite de fermentation avec différentes conditions d'OTR**

Les essais réalisés dans les exemples 1 et 2, sans limitation en sources nutritives, permettent d'établir un modèle par lequel il est possible de contrôler la production de lipides en respectant un $qO_2$ cible défini par l'équation 2 (qui vaut 27,4 mmoles d'$O_2$/L de culture et par /h - cf. exemple 2).

A partir ce $qO_2$ cible initial, des variations ont été testées.

Les niveaux de variation, de part et d'autre de ce qO2 cible, sont illustrés ici par l'OTR max en début de culture..

La valeur d'OTR max comprise entre 25 et 30 correspond à l'OUR optimale au début de culture définie dans le cadre de l'invention pour contrôler l'apport d'oxygène et afin de respecter le $qO_2$ cible.

Le tableau XI présente les paramètres de fermentation mesurés à T65 avec CNCM I-4702.

Tableau XI

| | | Concentrations | | | |
|---|---|---|---|---|---|
| % du $qO_2$ cible | OTR Max (mmole/L/h) au début de culture | Biomasse (g/L) | Acides Gras (g/100g) de biomasse | Acide palmitique (g/100 g de lipides) | DHA (g/100 g de lipides) |
| 60 | 15 | 41 | 41 | 36 | 48 |
| 75 | 20 | 52 | 41 | 36 | 46 |
| **100** | **25** | **57** | **47** | **39** | **45** |
| | **30** | **60** | **48** | **43** | **43** |
| 150 | 40 | 74 | 55 | 49 | 37 |
| 200 | 50 | 81 | 61 | 57 | 30 |

Ainsi, sur la base de ces résultats, on peut constater que, lorsque l'oxygène est apporté en excès par rapport à la valeur définie par l'équation 2, on observe une dilution du DHA dans les lipides de débordement, notamment l'acide palmitique.

A contrario, lorsque l'apport d'oxygène est suboptimal par rapport à la valeur définie par l'équation 2, la quantité de DHA produite est moindre.

**Exemple 4** : **exemple comparatif avec et sans limitation carence en azote.**

Les essais ont été réalisés avec :

- en Témoin : la souche CNCM I-4702, cultivée dans les conditions définies dans l'état de l'art constitué par la demande de brevet WO 01/54510, c'est-à-dire découplage de la phase de croissance et de production, elle-même induite lors de l'étape de limitation de l'apport en source azotée. Cette limitation a ici été induite par l'interruption de la régulation de pH avec l'ammoniaque à la fin du premier tiers de la culture.
  Afin de ne rendre compte que du comportement de la souche CNCM I-4702 quant à sa production de lipides dans des conditions de carence en azote, l'aération n'a pas été régulée en cascade, mais maintenue à une $pO_2$ supérieure à 10 %.
- une fermentation dans laquelle, pendant toute la durée de la culture, aucune limitation nutritionnelle n'a été imposée à la souche, et la $pO_2$ a été également régulée à 10 %.

Les résultats sont les suivants :

Tableau XII

| | | Pas de limitation nutritive | Limitation en azote |
|---|---|---|---|
| Biomasse | (g/L) | 76 | 86 |
| Acides Gras sur biomasse | (g/g) | 0,57 | 0,65 |
| Palmitique / AG | (g/g) | 0,67 | 0,62 |
| DHA / AG | (g/g) | 0,21 | 0,24 |

La richesse en acide gras de la biomasse n'est que très peu réduite sans limitation nutritive. Contrairement aux préjugés techniques en la matière, une limitation en source azotée n'est donc pas nécessaire pour induire la production de lipides.

**Exemple 5** : **exemple comparatif avec la souche ATCC 20888.**

Le protocole de culture utilisé est celui décrit dans l'exemple 4 de la demande de brevet WO 01/54510. La souche utilisée est *Schizochytrium sp* ATCC 20888.

Il est suivi l'enseignement de ladite demande, en comparaison des deux modes de conduite suivants :

1. Une régulation permanente supérieure à 10 %

2. Une $pO_2$ de 0% constante.

Les résultats obtenus sont présentés dans le tableau XIII suivant.

Tableau XIII

| | $pO_2$ 0% constant | $pO_2$ selon le protocole WO 01/54510 (8 - 4 - 0,5 à 0 %) | $pO_2$ 10 % constant |
|---|---|---|---|
| Temps (h) | 74 | 74 | 74 |
| AGPI / total acides gras (%) | **44,0** | **43,8** | **36,1** |
| DHA / total acides gras (%) | 33,6 | 32,8 | 27,1 |
| total acides gras (%) Biomasse | 45,9 | 44,6 | 44,8 |

(suite)

| | $pO_2$ 0% constant | $pO_2$ selon le protocole WO 01/54510 (8 - 4 - 0,5 à 0 %) | $pO_2$ 10 % constant |
|---|---|---|---|
| Biomasse totale (g/L) | 166 | 147 | 187 |
| DHA g/L/h | 0,35 | 0,29 | 0,31 |
| DHA % Biomasse | 15 | 15 | 12 |
| Inoculum (indicatif) g/L | 23 | 10,5 | 11 |

En suivant les enseignements de l'état de l'art, notamment ceux de la demande de brevet WO 01/54510, il est ainsi trouvé que, contrairement à ce qui est divulgué, une régulation constante de la $pO_2$ à 0 % ou à plus de 10 % pendant toute la durée de la fermentation permettait de produire plus de 35 % d'AGPI dans les acides gras, tout comme avec une conduite raisonnée avec une réduction de la $pO_2$ en cascade (10 %, puis 4 %, puis 0,5 % de saturation).

## Revendications

**1.** Procédé de production d'une biomasse de microalgues du genre *Thraustochytrium* enrichie en acide docosahexaénoïque (DHA) **caractérisé en ce que**, pendant la phase de culture en conditions hétérotrophiques, l'apport en oxygène est contrôlé de manière à satisfaire uniquement les besoins en oxygène 1) pour la production d'énergie nécessaire à la maintenance cellulaire, 2) pour la production des lipides de base, et 3) pour la croissance de la biomasse hors acides gras et **en ce que** l'apport en oxygène nécessaire pour satisfaire uniquement les besoins 1), 2) et 3) est calculé par l'équation 2 suivante

$$qO2Cible = (qm \times \frac{6\times32}{180}) + (qlipidesdebase \times y^{O2}/_{lipides}) + (\mu \times y^{O2}/_{x})$$

dans laquelle

$qO_2Cible$ est la quantité d'oxygène en gramme par gramme de biomasse hors acides gras et heure ;
qm est coefficient de maintenance exprimé en g de glucose par g de biomasse hors acides gras et par heure ;
$q_{lipides\ de\ base}$ est la vitesse d'accumulation des lipides de base exprimée en g de lipides de base par g de biomasse hors acides gras et par heure ;
$y^{O2}/_{lipides}$ est le coefficient de consommation d'oxygène par rapport à la formation de lipides exprimé en g d'oxygène par g de lipides,
$\mu$ est la vitesse de croissance exprimée en g de biomasse hors acides gras formée par g de biomasse hors acides gras et par heure, soit ($h^{-1}$) ;
$y^{O2}/_{x}$ est le coefficient de consommation d'oxygène par rapport à la formation de biomasse hors acides gras exprimé en g d'oxygène par g de biomasse hors acides gras.

**2.** Procédé selon l'une quelconque des revendications précédentes, **caractérisé en ce qu'**aucune limitation d'un élément nutritif, notamment en source carbonée ou azotée, n'est appliquée pendant le procédé fermentaire.

**3.** Procédé selon l'une quelconque des revendications précédentes, **caractérisé en ce que** les phases de croissance et de production de DHA sont concomitantes.

**4.** Procédé selon l'une quelconque des revendications précédentes, **caractérisé en ce que** les microalgues sont du genre *Schizochytrium sp ou Schizochytrium mangrovei.*

**5.** Procédé selon l'une quelconque des revendications précédentes, **caractérisé en ce que** les microalgues sont une souche sélectionnée parmi les souches CNCM I-4469 et CNCM I-4702 déposées à la Collection Nationale de Cultures de Microorganismes de l'Institut Pasteur respectivement le 14 avril 2011 et le 22 novembre 2012.

**6.** Procédé selon l'une quelconque des revendications précédentes, **caractérisé en ce qu'**il comprend en outre la récolte de la biomasse, éventuellement la préparation d'un extrait ou lysat cellulaire à partir de cette biomasse, puis facultativement l'extraction d'une huile brute riche en DHA.

**7.** Procédé selon l'une quelconque des revendications précédentes, **caractérisé en ce que** la biomasse obtenue comprend au minimum 40 % de DHA en poids d'acides gras totaux.

**8.** Procédé selon l'une quelconque des revendications précédentes, **caractérisé en ce que** la biomasse obtenue comprend au maximum 40 % d'acide palmitique en poids d'acides gras totaux.

**9.** Procédé selon l'une quelconque des revendications précédentes, **caractérisé en ce que** la biomasse obtenue comprend au minimum 25 % d'acides gras en sec poids de biomasse.

## Patentansprüche

**1.** Verfahren zur Herstellung einer Biomasse aus Mikroalgen der Gattung *Thraustochytrium* angereichert mit Docosahexaensäure (DHA), **dadurch gekennzeichnet, dass** während der Kulturphase unter heterotrophen Bedingungen die Sauerstoffzufuhr in einer Weise kontrolliert ist um nur die Bedürfnisse an Sauerstoff zu erfüllen 1) zur Erzeugung von Energie, die für die Zellerhaltung notwendig ist, 2) zur Herstellung von Basislipiden und 3) zum Wachstum der Biomasse außer Fettsäuren und dass die Sauerstoffzufuhr, die notwendig ist um nur die Bedürfnisse 1), 2) und 3) zu erfüllen, durch die folgende Gleichung 2 berechnet ist

$$\text{qO2Ziel} = (\text{qm x } \frac{6x32}{180}) + \text{qBasislipide x y}^{O2}/_{Lipide}) + (\mu \text{ x y } ^{O2}/_{x})$$

wobei

qO2Ziel die Sauerstoffmenge ist in Gramm pro Gramm Biomasse außer Fettsäuren und Stunde;
qm der Erhaltungskoeffizient ist, ausgedrückt in g Glukose pro g Biomasse außer Fettsäuren und pro Stunde;
$q_{Basislipide}$ die Akkumulationsgeschwindigkeit der Basislipide ist, ausgedrückt in g Basislipide pro g Biomasse außer Fettsäuren und pro Stunde;
$y^{O2}/_{Lipide}$ der Sauerstoffverbrauchskoeffizient im Vergleich zur Bildung von Lipiden ist, ausgedrückt in g Sauerstoff pro g Lipide,
$\mu$ die Wachstumsgeschwindigkeit ist, ausgedrückt in g Biomasse außer gebildete Fettsäuren pro g Biomasse außer Fettsäuren und pro Stunde, das heißt ($h^{-1}$);
$y\ ^{O2}/_{x}$ der Sauerstoffverbrauchskoeffizient im Vergleich zur Bildung von Biomasse außer Fettsäuren ist, ausgedrückt in g Sauerstoff pro g Biomasse außer Fettsäuren.

**2.** Verfahren nach einem der vorhergehenden Ansprüche, **dadurch gekennzeichnet, dass** während des Fermentationsprozesses keine Beschränkung eines Nährstoffelements, insbesondere einer Kohlenstoff- oder Stickstoffquelle, angewendet ist.

**3.** Verfahren nach einem der vorhergehenden Ansprüche, **dadurch gekennzeichnet, dass** die Wachstumsphase und die Phasen der DHA-Herstellung gleichzeitig sind.

**4.** Verfahren nach einem der vorhergehenden Ansprüche, **dadurch gekennzeichnet, dass** die Mikroalgen von der Gattung *Schizochytrium sp* oder *Schizochytrium mangrovei* sind.

**5.** Verfahren nach einem der vorhergehenden Ansprüche, **dadurch gekennzeichnet, dass** die Mikroalgen ein Stamm sind, ausgewählt aus den Stämmen CNCM I-4469 und CNCM I-4702, hinterlegt bei der Collection Nationale de Cultures de Microorganismes de l'Institut Pasteur am 14. April 2011 bzw. am 22. November 2012.

**6.** Verfahren nach einem der vorangehenden Ansprüche, **dadurch gekennzeichnet, dass** es ferner das Ernten der Biomasse, gegebenenfalls die Herstellung eines Zellextrakts oder Zelllysats aus dieser Biomasse, und dann fakultativ die Extraktion eines Rohöls, das reich an DHA ist, umfasst.

7. Verfahren nach einem der vorhergehenden Ansprüche, **dadurch gekennzeichnet, dass** die erhaltene Biomasse mindestens 40 % DHA, bezogen auf das Gewicht der gesamten Fettsäuren, umfasst.

8. Verfahren nach einem der vorhergehenden Ansprüche, **dadurch gekennzeichnet, dass** die erhaltene Biomasse höchstens 40 % Palmitinsäure, bezogen auf das Gewicht der gesamten Fettsäuren, umfasst.

9. Verfahren nach einem der vorhergehenden Ansprüche, **dadurch gekennzeichnet, dass** die erhaltene Biomasse mindestens 25 % Fettsäuren, bezogen auf das Trockengewicht der Biomasse umfasst.

## Claims

1. A method for producing a biomass of microalgae of the genus *Thraustochytrium* enriched in docosahexanoic acid (DHA), **characterized in that**, during the phase of culturing under heterotrophic conditions, the supply of oxygen is controlled so as to satisfy only the oxygen requirements 1) for energy production necessary for cell maintenance, 2) for base lipid production, and 3) for growth of the biomass apart from fatty acids and **in that** the supply of oxygen necessary to satisfy only the requirements 1), 2) and 3) is calculated by the following equation 2

$$qO2Cible = (qm \times \frac{6\times 32}{180}) + (qlipidesdebase \times y^{02}/_{lipides}) + (\mu \times y^{O2}/_{x})$$

in which

$qO_2$ Target is the amount of oxygen in grams per gram of biomass apart from fatty acids and per hour;
qm is the coefficient of maintenance expressed in g of glucose per g of biomass apart from fatty acids and per hour;
$q_{base\ lipids}$ is the rate of accumulation of base lipids expressed in g of base lipids per g of biomass apart from fatty acids and per hour;
$y^{O2}/_{lipids}$ is the coefficient of oxygen consumption relative to lipid formation expressed in g of oxygen per g of lipids ;
$\mu$ is the rate of growth expressed in g of biomass formed apart from fatty acids per g of biomass apart from fatty acids and per hour, or ($h^{-1}$) ;
$y^{O2}/_{x}$ is the coefficient of oxygen consumption relative to biomass formation apart from fatty acids expressed in g of oxygen per g of biomass apart from fatty acids.

2. The method as claimed in any one of the preceding claims, **characterized in that** no nutritional element, especially a carbon or nitrogen source, is limited during the fermenting method.

3. The method as claimed in any one of the preceding claims, **characterized in that** the phases of growth and production of DHA are concomitant.

4. The method as claimed in any one of the preceding claims, **characterized in that** the microalgae are of the genus *Schizochytrium sp or Schizochytrium mangrovei.*

5. The method as claimed in any one of the preceding claims, **characterized in that** the microalgae are a strain selected from the strains CNCM I-4469 and CNCM I-4702 deposited with the Collection Nationale de Cultures de Microorganismes (French National Collection of Microorganism Cultures) of the Institut Pasteur on April 14, 2011 and November 22, 2012, respectively.

6. The method as claimed in any one of the preceding claims, **characterized in that** it also comprises harvesting the biomass, optionally preparing a cell extract or a lysate from this biomass, then optionally extracting a DHA-rich crude oil.

7. The method as claimed in any one of the preceding claims, **characterized in that** the biomass obtained comprises at least 40% of DHA by weight of total fatty acids.

8. The method as claimed in any one of the preceding claims, **characterized in that** the biomass obtained comprises at most 40% of palmitic acid by weight of total fatty acids.

9. The method as claimed in any one of the preceding claims, **characterized in that** the biomass obtained comprises at least 25% of fatty acids by dry weight of biomass.

# FIGURE 1

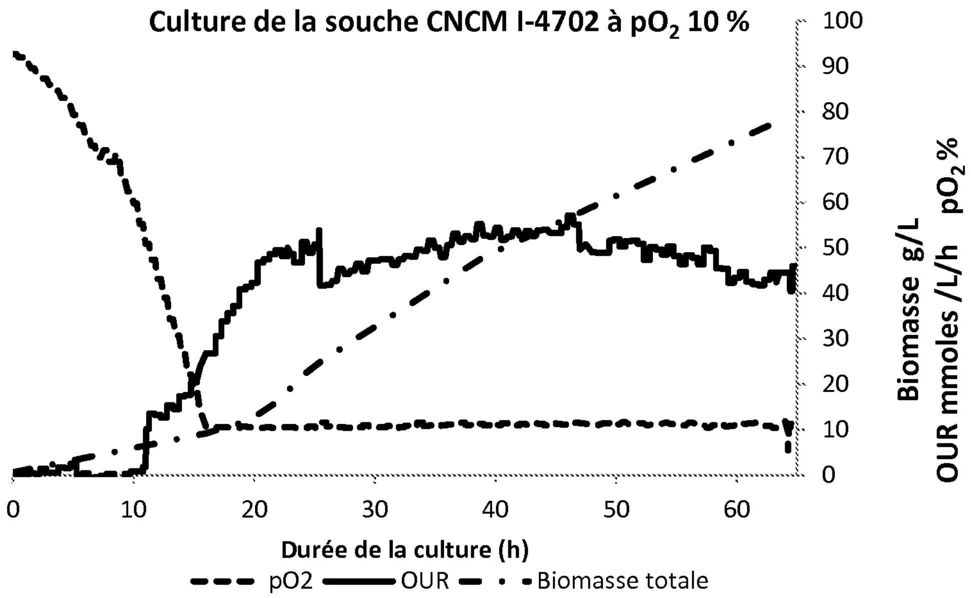


# FIGURE 2

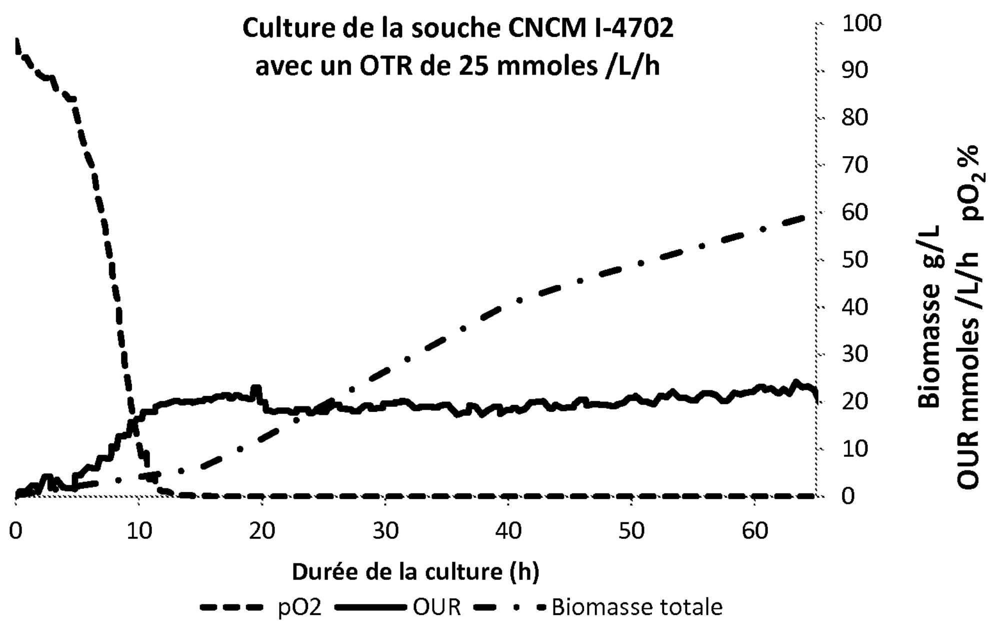

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0154510 A


### Littérature non-brevet citée dans la description


- **JIANG ; CHEN.** *Process Biochem.,* 2000, vol. 35 (10), 1205-1209

SEQUENCE LISTING

<110> ROQUETTE FRERES

<120> PROCEDE D'ENRICHISSEMENT EN DHA DE LA BIOMASSE DE MICROALGUES DU GENRE TRAUSTOCHYTRIUM

<130> B1734PC

<160> 2

<170> PatentIn version 3.3

<210> 1
<211> 479
<212> DNA
<213> Schizochytrium sp

<400> 1

```
gagggtttta cattgctctc attccaatag caagacgcga agcgccccgc attgatattt      60
ctcgtcacta cctcgtggag tccacattgg gtaatttacg cgcctgctgc cttccttgga     120
tgtggtagcc gtctctcagg ctccctctcc ggagtcgagc cctaactccc cgtcacccgt     180
tatagtcacc gtaggccaat accctaccgt cgacaactga tggggcagaa actcaaacga     240
ttcatcgctc cgaaaagcga tctgctcaat tatcatgact caccaagaga gttggcttag     300
acctaataag tgcggccctc cccgaaagtc gggcccgtac agcacgtatt aattccagaa     360
ttactgcagg tatccgtata aaggaactac cgaagggatt ataactgata taatgagccg     420
ttcgcagttt cacagtataa ttcgcttata cttacacatg catggcttag tctttgaga      479
```

<210> 2
<211> 454
<212> DNA
<213> Schizochytrium mangrovei

<400> 2

```
ggttttacat tgctctcatt ccgatagcaa aacgcataca cgcttcgcat cgatatttct     60
cgtcctacct cgtggagtcc acagtgggta atttacgcgc ctgctgctat ccttggatat    120
ggtagccgtc tctcaggctc cctctccgga gtcgagccct aactctccgt cacccgttat    180
agtcaccgta gtccaataca ctaccgtcga caactgatgg ggcagaaact caaacgattc    240
atcgaccaaa awagtcaatc tgctcaatta tcatgattca ccaataaaat cggcttcaat    300
ctaataagtg cagccccata cagggctctt acagcatgta ttatttccag aattactgca    360
ggtatccata taaaagaaac taccgaagaa attattactg atataatgag ccgttcgcag    420
tctcacagta caatcgctta tacttacaca gcag                                454
```